# EUROPEAN PATENT APPLICATION

(11) **EP 4 062 879 A1**
(43) Date of publication of application: **28.09.2022**
(21) Application number: 21165408.2
(22) Date of filing: 26.03.2021
(51) Int. Cl.: A61F 5/00

(54) **GASTRO-INTESTINAL TUBE AND ANCHORING THEREFOR**

(71) Applicant: BariaTek Medical, 75003 Paris (FR)
(72) Inventor: BIADILLAH, Joussef, 78600 Maisons-Laffitte (FR)
(74) Representative: Hepp Wenger Ryffel AG

(57) **Abstract**

An anchor (20) for anchoring a tube (10) with respect to a pylorus of a patient. The tube (10) may be a bypass tube or bypass conduit for bypassing a portion of the bowel. The anchor (20) may comprise a gastric bulb (22) collapsible for introduction and optionally self-expanding to an operative condition. The bulb (22) may have an open structure of a low pressure chamber. The bulb (22) may be configured to partly collapse in response to stomach contractions. This enables the bulb to exhibit compliance with the stomach contractions, and absorb the contractions. Other embodiments of tubes, anchors, balloons, and stapling, are also described.

## Description

The present invention relates to the field of tubes for insertion into the gastro-intestinal tract, and to anchors therefor. In some non-limiting aspects, the tubes are bypass tubes for bypassing portions of the bowel.

Various surgical techniques, and implants, have been proposed for treating obesity and diabetes. Surgical techniques include creation of gastric pockets and gastric bypasses of the stomach, duodenum and part of the jejunum. Bypass tubes or liners have been proposed for insertion into the gastro-intestinal tract, to bypass the duodenum and optionally part of the jejunum.

Technical challenges remain for many of these techniques. For example, the endoscopic placement and anchoring of bypass tubes remains challenging. One technique proposed is to anchor the tube in the vicinity of the pylorus. However, the stomach and intestine are subject to significant motion in normal bodily function. Muscular contractions of the stomach, including at the pyloric antrum, complicate maintaining the tube in position. The muscular contractions can be extreme in the case of, for example, the patient vomiting. Dislodgement either towards the duodenum, or into the stomach, can necessitate medical intervention to correct the position or to retrieve the bypass tube. Many existing proposals are compromised by the apparently conflicting need for secure anchoring, yet with atraumatic engagement with the body tissue to avoid the device causing tissue irritation.

It would be desirable to address and/or mitigate one or more of the above issues.

Aspects of the invention are defined in the claims.

A first aspect of the disclosure provides an anchor for anchoring a tube with respect to a pylorus of a patient. The tube may be a bypass tube or bypass conduit for bypassing a portion of the bowel.

The anchor may comprise a gastric bulb coupled or couplable to a proximal end of the tube. The gastric bulb may be expandable from a collapsed condition for introduction into the stomach, to an operative condition within the stomach.

In some embodiments, the bulb may be configured such that, in the expanded condition a pressure within the bulb is (i) not greater than atmospheric pressure, and/or (ii) not greater than surrounding pressure within the stomach. With such an arrangement, the bulb is not permanently substantially pressurized compared to the surrounding ambient pressure within the stomach.

The gastric bulb may be configured to partly collapse in response to stomach contractions. This enables the bulb to exhibit compliance with the stomach contractions, and absorb the contractions without applying significant pulling force on the tube, which might otherwise dislodge the tube.

In some embodiments, the gastric bulb is self-expandable from the collapsed condition to the operative condition. The gastric bulb may comprise a self-expandable structure, optionally a frame structure (e.g. with ribs and/or struts), and/or a mesh structure.

The gastric bulb may be open and/or have communication apertures allowing chyme to pass into the interior of the bulb. For example, the bulb may comprise an open frame or mesh. Alternatively, the bulb may comprise a fluid-tight chamber. The chamber may have an inlet port that may: (i) allow fluid (e.g. gas, such as air) to be drawn into the chamber as the gastric bulb expands to its operative condition (e.g. in embodiments in which the pressure within the bulb is intended not to be greater than atmospheric pressure, and/or not greater than surrounding pressure within the stomach); and/or (ii) admitting fluid into the chamber for inflating the gastric bulb (e.g. in embodiments using a pressurized balloon).

Additionally or alternatively to any of the above, the gastric bulb may be spaced from the proximal end of the tube. Optionally, the gastric bulb is coupled physically to the tube by one or more struts or flexible tethers. Alternatively, the gastric bulb is magnetically attracted or repelled by the tube, such that there is magnetic coupling or magnetic interaction between the gastric bulb and the proximal end of the tube.

The bulb may have any desired shape in the operative condition, for example, generally spherical, teardrop shaped, lozenge shaped, rugby-ball shaped, bell shaped (or tulip shaped).

Additionally or alternatively to any of the above, in a second aspect, the anchor may comprise at least one resilient element, expandable from a collapsed condition for introduction into the stomach, and an expanded operative condition in which the resilient element has an at least partly helix shape.

The resilient member may optionally define a helix shape that expands in a radial direction progressively away from the proximal end of the tube. The helix shape may include open space between adjacent turns or windings of the helix.

Such a configuration may be advantageous to be easily collapsed for introduction, while also expanding to a relatively large size in its operative condition.

Additionally or alternatively to any of the above, a third aspect of the disclosure provides an anchor for anchoring a tube with respect to a pylorus of a patient, the anchor comprising a first ring for fitting against a stomach-side of a pyloric sphincter, and a second ring for fitting against a duodenal-side of the pyloric sphincter. The first and second rings are coupled to one another for clamping the pyloric sphincter between the rings, the first and second rings having different inner and/or outer diameters from each other, optionally such that one ring has an inner diameter greater than the outer diameter of the other.

Such an anchor can attach firmly, yet atraumatically, to the pyloric sphincter, providing good fixation even in the presence of strong stomach contractions.

The first and/or second rings may be collapsible to a collapsed condition for introduction to the pylorus. The anchor may further comprise connecting elements between the rings, and coupled to the rings for selectively drawing the rings closer to one another for clamping the pyloric sphincter, the connecting elements optionally being sutures.

In some embodiments, the anchor further comprises at least one inner ring for corralling an inner peripheral edge of the pyloric sphincter, optionally wherein the at least one inner ring comprises first and second inner rings.

The first and/or second ring may be made of, or comprise, any suitable material, for example one or more of: metal; and/or a shape memory alloy optionally, nitinol; and/or plastics.

Additionally or alternatively to any of the above, a fourth aspect of the disclosure provides a tube for insertion into at least the duodenum of a patient, and at least one gastric bulb for inducing, via contact with the stomach wall, a feeling of satiety. Various techniques are envisaged for configuring the bulb to contact the stomach wall, for example, one or any combination of the following:
- The bulb may optionally be inflated with, for example, air or gas allowing the bulb to float in the stomach.
- Multiple bulbs may be provided to at least partly fill the stomach while allowing normal stomach movements and contractions.
- The bulb may be repelled from the tube, for example, by magnetic repulsion, in a direction towards the stomach wall.

Additionally or alternatively to any of the above, a fifth aspect of the disclosure provides a tube for introduction into a gastro-intestinal tract of a patient, the gastro-intestinal tract optionally being a modified tract (for example, following bypass surgery that modifies the digestive route). The tube may be configured for introduction at an anastomosis.

The tube is configured for admitting passage of stomach and/or bowel content in a first direction therethrough, and for obstructing passage of stomach and/or bowel content in an opposite second direction.

For example, the tube may comprise at least one, optionally a plurality, of valving elements configured for admitting passage of stomach and/or bowel content in the first direction therethrough, and for obstructing passage of stomach and/or bowel content in the second direction. The valving element may comprise a flap that opens to admit passage stomach and/or bowel content in the first direction through the tube, and closes to obstruct passage of stomach and/or bowel content in the second direction.

Such a tube may be advantageous in obstructing reflux of digestive juices towards the stomach and/or the oesophagus. For example, the tube may obstruct digestive juices from the pancreas from passing against the usual flow direction through an anastomosis. This can reduce discomfort that may be experienced by the patient following surgery.

Additionally or alternatively to any of the above, a sixth aspect of the disclosure provides a tube for introduction at the duodenum of a patient, the tube made of plastics and comprising a suture-permeable and/or staple-permeable layer of woven or non-woven fabric near or at the proximal end. The fabric permits anchoring of the tube by suturing or stapling through the fabric into body tissue near or at the pylorus. The fabric may be laminated to, or incorporated in, the plastics of the tube. The fabric serves to prevent propagation of tears that otherwise occur in plastics material, especially when subjected to stomach movements and contractions.

Additionally or alternatively to any of the above, a seventh aspect of the disclosure provides a tube for introduction into a gastro-intestinal tract of a patient, the outer surface of the tube being provided with an open channel extending at least partly axially and/or longitudinally with respect to the tube. The channel permits gastric secretions, for example, pancreatic juices, to travel outside the tube, and avoids the secretions being trapped by contact between the tube and the bowel wall. Optionally, the channel may have a helical shape around the exterior of the tube. Alternatively, the channel may be a longitudinal flute. More than one channel may be provided.

Additionally or alternatively to any of the above, an eighth aspect of the disclosure provides a technique for treating diabetes or obesity, by application of at least one tissue-penetrating fixing to reduce the natural volume of the stomach by creating one or more pleats (or artificial folds) in the stomach wall.

As used herein, the term "tissue-penetrating fixing" includes any fixing that penetrates, optionally penetrates entirely through, tissue, and includes (at least) a suture, a staple or a tag-pin. The tissue-penetrating fixing may, for example, be placed by an endoscopic technique or a laparoscopic technique.

The one or more pleats may extend in a direction that is generally (i) from top to bottom of the stomach, and/or (ii) from the oesophagus to the pylorus. Such pleats can form a generally sleeve shape cavity or passage in the stomach.

The eighth aspect may optionally be used with a bypass tube and/or an anastomosis, for increasing the effectiveness of such devices by reducing stomach volume in an efficient manner. For example, the eighth aspect can in particular be used with any of the other devices described herein.

In all of the above aspects, the tube may be a bypass tube or bypass conduit for bypassing a portion of the bowel. Non-limiting embodiments of the invention are now described, by way of example only, with reference to the accompanying drawings, in which:
Fig. 1 is a schematic side view of a first example of a gastric anchor for a duodenal tube.
Fig. 2 is a schematic section illustrating the first example in an implanted condition.
Fig. 3 is a schematic side view if a second example of a gastric anchor similar to Fig. 1.
Fig. 4 is a schematic section illustrating in an implanted condition a further example similar to the first and second examples.
Fig. 5 is a schematic section illustrating in an implanted condition a further example having at least one balloon.
Fig. 6 is a schematic section illustrating in an implanted condition a further example having at least one balloon.
Fig. 7 is a schematic section illustrating in an implanted condition a further example having multiple balloons.
Fig. 8 is a schematic section illustrating a further example of an anchor for anchoring at the pylorus.
Fig. 9 is a schematic section illustrating a detail of Fig. 8.
Fig. 10 is a schematic section illustrating a further example of an anchor similar to that of Figs. 8 and 9.
Fig. 11 is a schematic side view of a further example of tube attachable by sutures and/or staples.
Figs. 12a-d are schematic sections illustrating steps for attaching a tube to a stomach wall with pledgets.
Figs 13a and 13b are schematic perspective views of an exterior profile of a tube in a further example.
Fig. 14 is a schematic view of a tube placed at a gastro-jejunal anastomosis.
Fig. 15 is a schematic section of the tube of Fig. 14 in isolation.
Fig. 16 is a schematic section illustrating formation of pleats to reduce stomach volume.
Fig. 17 is a schematic section illustrating a detail of Fig. 16.
Fig. 18 is a schematic section illustrating the stomach pleats in combination with other treatment devices for diverting flow of stomach contents.

In the following description, the same reference numerals are used to denote similar or equivalent features, whether or not described in detail. The description of one embodiment can thus be combined with another embodiment. Where a tube is described, the tube may optionally be a bypass tube or bypass conduit for bypassing a portion of the bowel to reduce nutrient uptake.

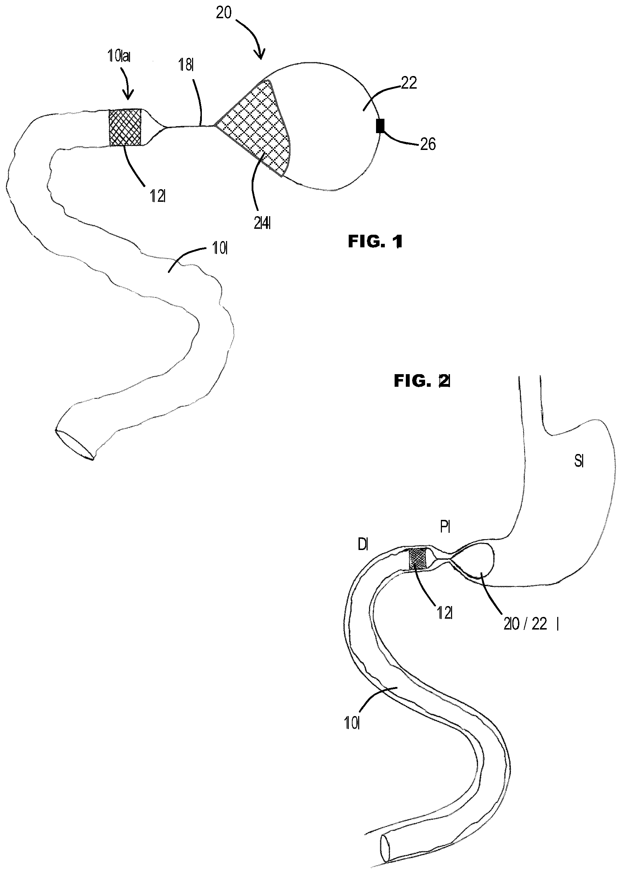

Referring to Figs. 1 and 2, a tube 10 is illustrated for introduction into at least the duodenum D of a patient, for the treatment of diabetes or obesity. The tube 10 optionally may extend to the jejunum. A proximal end 10a of the tube may be disposed in the duodenum D close to the pylorus P as shown in Fig. 2, or it may extend at least partly through the pylorus P. The tube 10 may optionally comprise a cuff or stent 12 for resisting displacement from the implanted position towards the stomach. The tube 10 may optionally comprise reinforcement (not shown in Figs 1 and 2, but reference 14 in later figures) for preventing twisting of the tube 10 at the pylorus that could create a blockage.

An anchor 20 for the tube 10 comprises a gastric bulb 22 for placement with the stomach S. The gastric bulb 22 has a collapsed state suitable for introduction into the stomach, for example, through the patient's mouth, and an operative state in which the bulb 22 bulges to define an anchor that resists displacement through the pylorus P.

The anchor 20 is coupled, or couplable, to the tube 10 by any suitable coupling, for example, by one or struts or one or more flexible tethers 18.

In the illustrated examples, the bulb 22 is configured such that, in the expanded condition a pressure within the bulb is (i) not greater than atmospheric pressure, and/or (ii) not greater than surrounding pressure within the stomach. The bulb 22 is not permanently substantially pressurized compared to the surrounding ambient pressure within the stomach. This contrasts with a type of balloon or bulb that is inflated, and distends from within by virtue of inflation pressure.

By virtue of, for example, absence of a positive pressure within the bulb 22, the bulb 22 is able to partly collapse in response to stomach contractions. This enables the bulb 22 to exhibit, at least partly, compliance with the stomach contractions, and to absorb the contractions without pulling overly on the tube 10, which might otherwise dislodge the tube 10 into the stomach. The bulb 22 can partly compress to absorb or accommodate extreme transient stomach contractions, for example, should the patient vomit. At the same time, the bulb 22 is biased to its operative condition in which is serves to prevent displacement in the distal direction, as the bulb 22 will resist passage towards and through the pylorus.

In the illustrated examples, the bulb 22 is self-expandable from the collapsed condition to the operative condition. The gastric bulb 22 can comprise a self-expandable structure 24 (depicted schematically in part in Fig. 2), optionally a frame structure (e.g. with ribs and/or struts), and/or a mesh structure.

In one version, the gastric bulb 22 may be open to its interior and/or have communication apertures allowing chyme to pass into the interior of the bulb 22. For example, the bulb 22 may comprise an open frame 24.

Alternatively, the bulb 22 may comprise a fluid-tight chamber. The chamber may have an inlet port 26 for allowing fluid to be drawn into the chamber as the gastric bulb 22 expands to its operative condition under the influence of the self-expanding frame 24. A delivery device may include a vent channel removably coupled to the inlet port 26 to allow pressure equalization. After pressure equalization is complete, the vent channel can be disconnected from the inlet port 26, thus sealing the chamber. The small quantity of fluid in the chamber can provide a compliant cushion that supplements the self-expanding frame 24, providing an atraumatic anchor with compliant characteristics. Optionally, the fluid-tight chamber may include a central passage (not shown) for allowing chyme to pass towards the pylorus and the entrance to the tube 10.

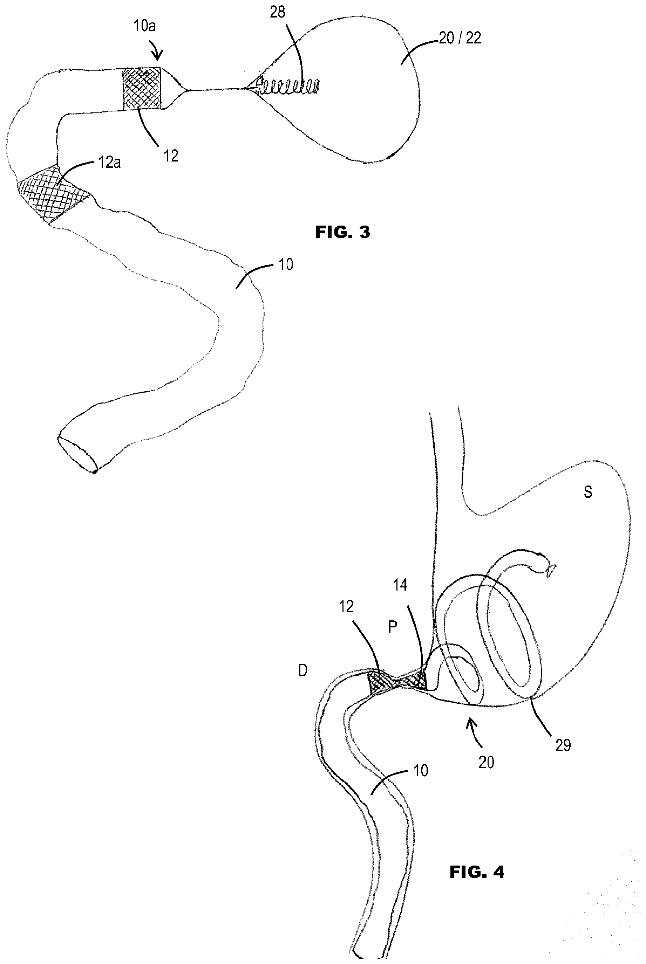

Fig. 3 illustrates a second example similar to the first example of Figs. 1 and 2. The main difference in Fig. 3 is the addition of an adjustment mechanism 28 for enabling the distance between the bulb 22 and the proximal end 10a of the tube 10 to be set prior to introduction into the stomach and/or in situ during or after placement in the stomach. The adjustment mechanism 28 may, for example, comprise a screw thread and a rotatable nut. The adjustment mechanism 28 may be adjusted may manual rotation using a suitable tool, or by magnetic rotation using a magnetic tool.

Referring to Fig. 4, an alternative example of anchor 20 is illustrated comprising at least one resilient element 29, expandable from a collapsed condition for introduction into the stomach, and an expanded operative condition (Fig. 4) in which the resilient element 30 has an at least partly helix shape.

The helix shape may diverge in a radial direction progressively away from the proximal end of the tube. The helix shape may include open space between adjacent turns or windings of the helix.

Such a configuration may be advantageous to be easily collapsed for introduction, while also expanding to a relatively large size in its operative condition. The helix shape can provide atraumatic compliant engagement able to absorb and accommodate stomach contractions, even strong transient contractions, without pulling overly on the tube 10. At the same time, the helix shape reliably anchors the tube 10 to prevent displacement through the pylorus. The anchor 20 can be collapsed by pulling on the free end of the helix shape using a suitable retrieval tool, allowing the device to be removed when desired.

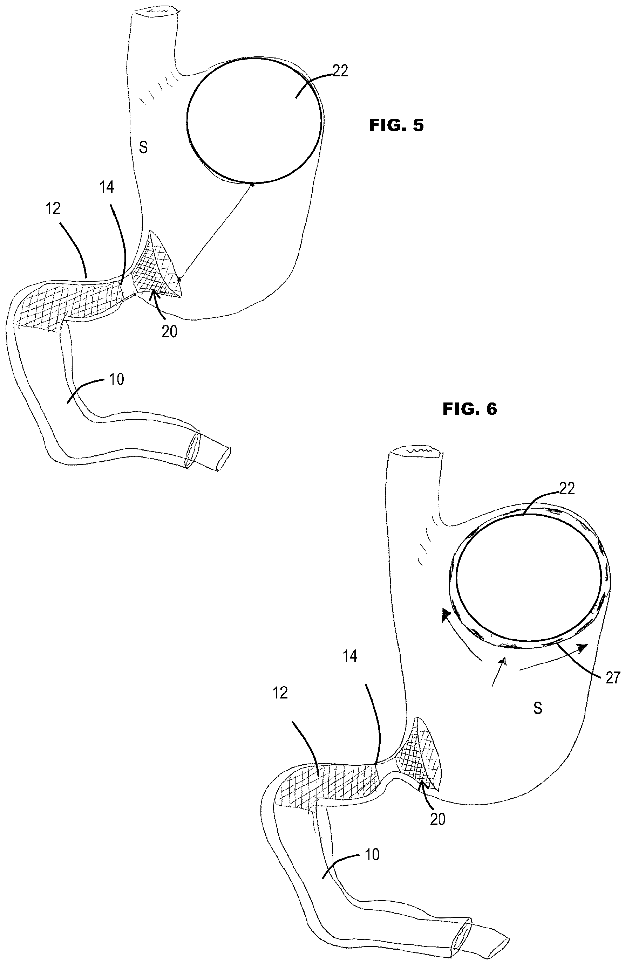

Figs. 5 to 7 illustrate further examples including one or more gastric bulbs or balloons 22 configured to induce a feeling of satiety through contact with the stomach wall. The balloons may or may not function as gastric anchors, depending on the implementation. Where appropriate a gastric anchor 20 is coupled to the proximal end of the tube 10.

The bulbs or balloons 22 may optionally be inflated with fluid (e.g. gas or liquid). A gas, for example air, may desirably cause the balloon to float with respect to stomach contents. In Fig. 5, at least one (optionally more) balloon 22 is tethered to the proximal end of the tube 10, to float upwardly with respect to the stomach contents.

In Fig. 6, at least one (optionally more) balloon 22 has a magnetic property, such as a magnetic coating or carries one or more magnetic elements 27. Magnetic repulsion between the balloon 22 and a same-sense magnetic element (not shown) at the proximal end of the tube 10 urges the balloon 22 away from the tube 10, and towards contact with the stomach wall.

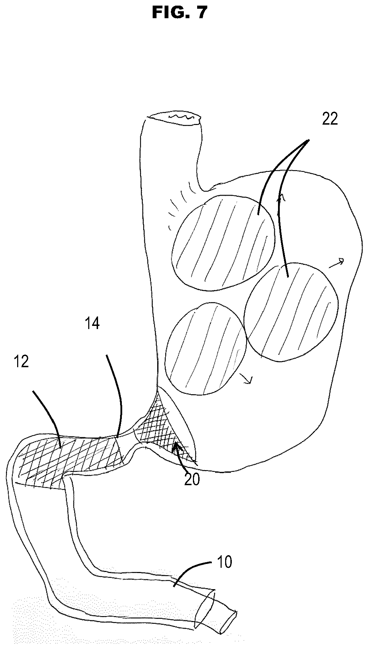

In Fig. 7, multiple balloons 22 occupy significant space within the stomach, and bear against the stomach wall(s). The balloons 22 may be loose and/or captive relative to one another. The balloons 22 may be loose and/or captive with respect to the tube 10.

In the above examples of Figs. 5 to 7, the one or more balloons 22 are sized to permit stomach movements and contractions, and also to permit chyme to pass to the tube 10.

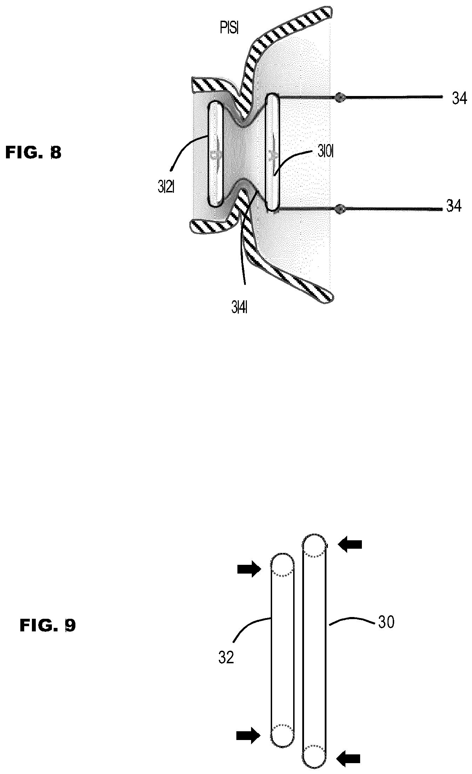

Referring to Figs. 8 to 10, the anchor 20 comprises a first ring 30 for fitting against a stomach-side of a pyloric sphincter PS, and a second ring 32 for fitting against a duodenal-side of the pyloric sphincter PS. The first and second rings 30, 32 are coupled to one another for clamping the pyloric sphincter PS between the rings 30 and 32. As can be seen in the detail of Fig. 9, the first and second rings 30 and 32 have different inner and/or outer diameters from each other. In the illustrated form, one ring (e.g. the first ring 30) has an inner diameter approximately greater than (or at least not substantially smaller than) the outer diameter of the other (e.g. the second ring 32).

Such an anchor 20 firmly, yet atraumatically, attaches to the pyloric sphincter PS, providing good fixation even in the presence of strong stomach contractions. In particular, the offsetting of one ring with respect to another can reduce risk of tissue necrosis that could be caused by pinching between two identical diameter rings with the same clamping force.

The first and/or second rings 30 and 32 may be collapsible to a collapsed condition for introduction to the pylorus. The anchor 20 further comprises one of more connecting elements 34 between the rings 30 and 32, and coupled to the rings 30 and 32 for selectively drawing the rings closer to one another for clamping the pyloric sphincter PS. In the illustrated form, the connecting elements 34 comprise filaments (e.g. sutures). The filaments engage the rings 30 and 32 such that pulling on the filaments draws at least one ring towards the other.

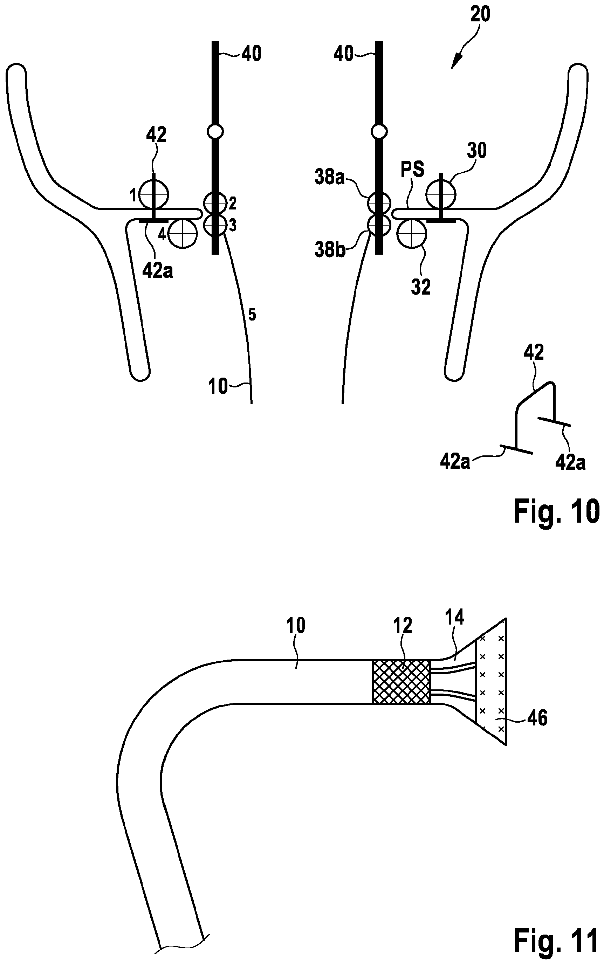

If desired, at least one of the rings 30, 32 may also be directly attached to the pylorus, for example, by one or more tissue-penetrating fixings (similar to that shown in Fig. 10, described below).

The tube (not shown) may be attached to one or more of the first and second rings 30 and 32. The tube may be attached permanently, or via a connector, such as a magnetic connector.

Referring to Fig. 10, in addition to the first and second rings 30 and 32, the anchor 20 further comprises at least one inner ring 38 for corralling an inner peripheral edge of the pyloric sphincter PS. In the illustrated embodiment, first and second inner rings 38a and 38b are used. One of more connecting elements 40 between the inner rings 38a and 38b, and coupled to the rings 38a and 38b selectively draw the rings closer to one another for corralling the inner edge of the pyloric sphincter PS. In the illustrated form, the connecting elements 40 comprise filaments (e.g. sutures). The filaments engage the rings 38a and 38b such that pulling on the filaments draws at least one ring towards the other.

In use, the after placing the second ring 32, the inner rings 38a and 38b may be placed spaced apart from the pyloric sphincter PS. The inner rings 38a and 38b are drawn together to corral the inner edge, followed by placing and/or drawing the first ring 30 to clamp the pylorus between the first and second rings 30 and 32. If desired, one (or both) of the first and second rings 30, 32 may be directly attached to the tissue of the pyloric sphincter, for example, by one or more tissue penetrating fixings 42. In one example, the fixings 42 are staples, for example having a U-shaped form, each limb of the U-shape piercing tissue and held captive by a transverse stop 42a (e.g. defining a T-shape). Alternatively, the fixings 42 may be tag-pins inserted from one side of the tissue, and having a self-expanding transverse stop or wing extending from a central stalk, to hold the fixing captive on the opposite side of the tissue, preventing withdrawal from the first side.

The tube 10 may optionally be attached (e.g. permanently or via an assemblable connection) to one of the rings, for example, one of the inner rings 38a, 38b, as illustrated.

Referring to Fig. 11, a further example of tube 10 is illustrated. The tube 10 is configured for extending from a proximal end 10a at the pylorus into the duodenum and optionally at least partly into the jejunum. The tube 10 may be made of plastics, such as silicone or polyurethane. The proximal end 10a is configured for direct attachment to body tissue by means of sutures or staples 42 that pierce the material of the tube 10. In order to prevent tearing of the plastics material, the proximal end comprises a layer of suture-permeable and/or staple-permeable, reinforcing fabric 46, optionally laminated to and/or incorporated within the plastics material. The fabric 46 may be a woven or non-woven material. The fabric 46 may be of polymer material, such as PET. The natural openings or spaces in the fabric material 46 allow a suture or fixing to pass therethrough without breaking the fiber or filament of the fabric itself. Thus the fabric can prevent tear propagation in the plastics of the tube 10.

The proximal end 10a of the tube 10 may be flared to facilitate placement and attachment of the proximal end 10a.

Optionally, the tube 10 includes a duodenal anchor 12, such as a stent, and/or reinforcement 14 across the pylorus to avoid risk of the tube 10 twisting due to stomach movements.

In use, the tube 10 may be placed into the duodenum and optionally the jejunum. A surgical stapler may be used to staple the proximal end to, for example, tissue forming the pyloric sphincter, in a similar manner to that illustrated in Fig. 10.

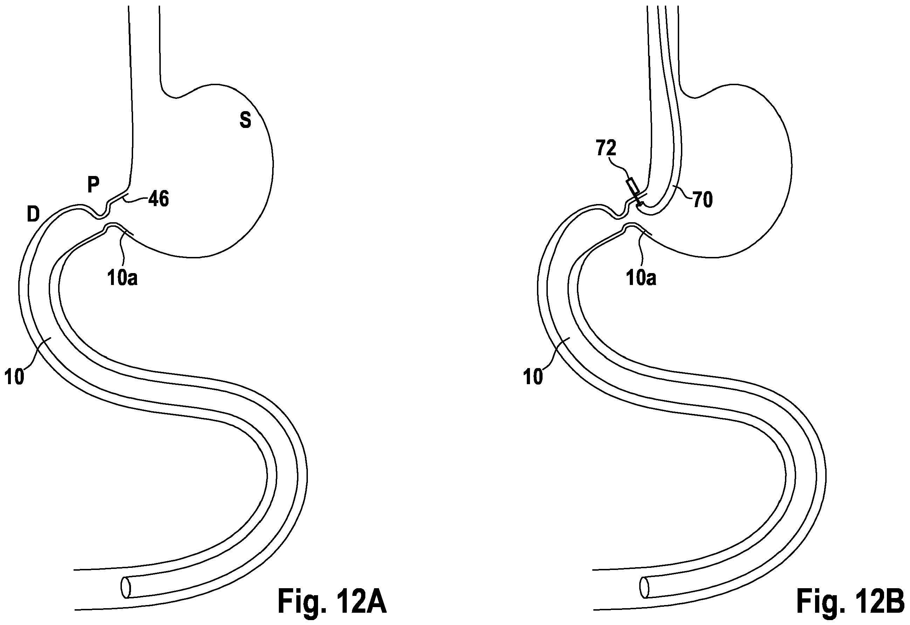

Figs. 12a to 12d illustrate a further technique for fastening the tube 10, such as the tube of Fig. 11. Referring to Fig. 12a, the tube 10 is introduced via the patient's mouth, and placed into at least the duodenum, with the proximal end 10a of the tube 10 proximal of the pylorus. The tube 10 may be held in place temporarily by a mechanical device (not shown).

Referring to Fig. 12b, a tool 70 is guided through the mouth to the pyloric antrum. The tool 70 includes a hypotube needle used to pierce through the region of the tube containing the fabric 46, and through the stomach wall. A pledget 72 having an attached suture is inserted through the fabric 46 and the stomach wall, via the hypotube needle, and deployed outside the stomach wall, leaving the suture trailing through the stomach wall and the fabric 46.

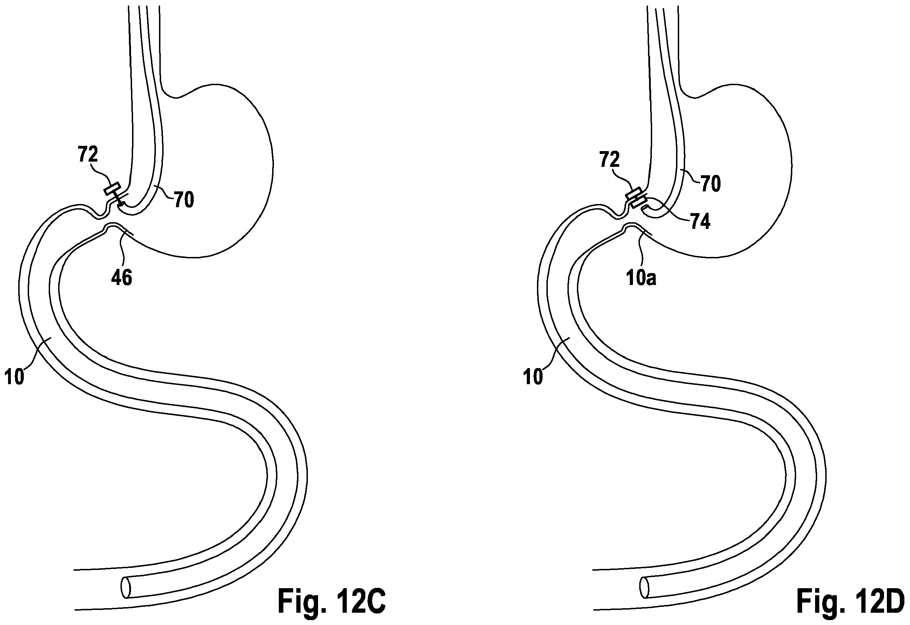

Referring to Figs. 12c and d, the hypotube needle is withdrawn to the interior side of the stomach, and a second pledget 74 is parachuted over the same suture, via the hypotube, and deployed on the interior surface of the stomach wall. The suture is tightened to tightly sandwich the stomach wall and the fabric 46 of the proximal end of the tube, between the two pledgets 72 and 74. The suture is knotted and cut, leaving the pledgets 72 and 74 forming an independent fastening. The same process is repeated at several different points around the periphery of the proximal end of the tube 10 to firmly anchor the tube 10 to the stomach wall. The pledgets can provide a secure attachment of the tube 10 and avoid irritation through rubbing during stomach contractions, because the proximal end of the tube is attached to move intimately with the stomach wall. When it is desired subsequently to remove the tube 10 from the stomach, a tool can be inserted into the stomach to cut the sutures, and release the pledgets 74 on the inside surface of the stomach wall, thereby releasing the tube 10.

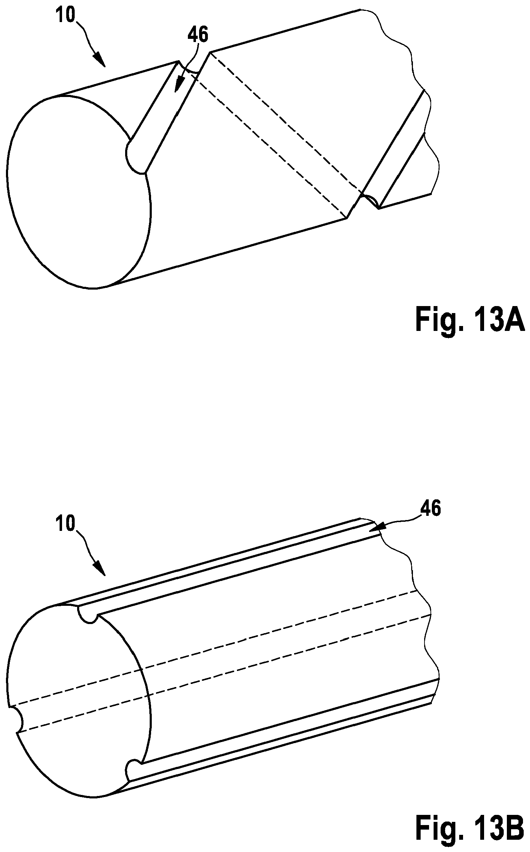

Referring to Figs. 13a and 13b, a tube 10 for insertion into the gastro-intestinal tract, optionally the duodenum, has an outer surface defining at least one channel 46 extending, at least partly, in an axial direction of the tube. In one form, the channel 46 may be substantially axial, for example, in the form of a fluted surface (Fig. 13b). Alternatively, the channel may be helical in shape, in the form of a threaded surface (Fig. 13a). The channel 46 permits bowel juices, for example, pancreatic juices, to flow as in the efferent bowel, along the exterior of the tube 10, and can avoid the juices becoming trapped by contact between the tube 10 and the bowel tissue.

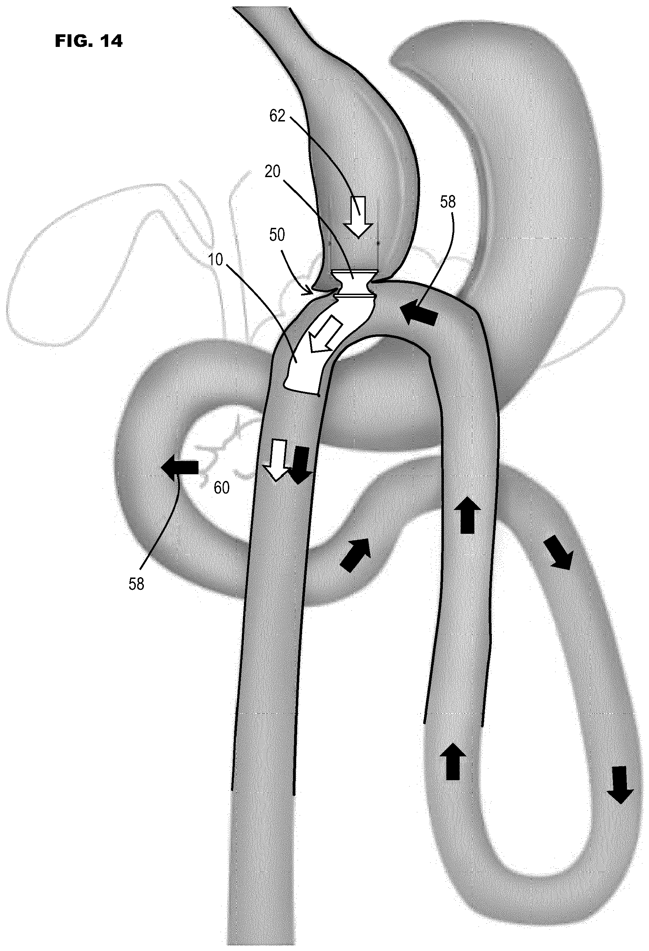

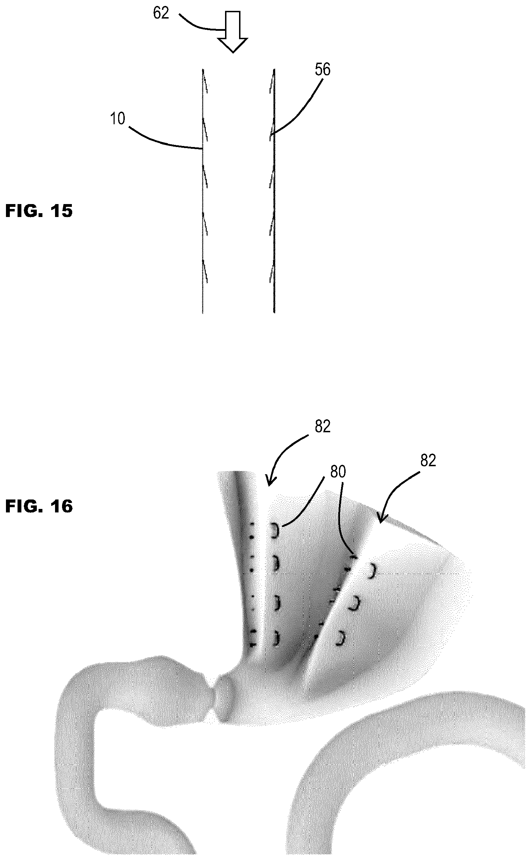

Referring to Figs. 14 and 15, a tube 10 is illustrated for supplementing an anastomosis 50, for example a gastro-jejunal anastomosis. The anastomosis may, for example, be between the bottom of the stomach and the jejunum, or it may be between a cutdown stomach and the jejunum (as illustrated in Fig. 14). The tube 10 may optionally comprise an anchor 20 for anchoring at the anastomosis 50. The anchor may include any of the features described above; optionally the anchor may include the features of any of Figs. 8 to 10.

The tube 10 may function to prevent or reduce reflux of pancreatic juices (illustrated by arrows 58) originating from the pancreas 60 from passing through the anastomosis 50. For example, the tube 10 is placed into the part of the jejunum leading to the large intestine. Stomach contents (arrows 60) passing through the anastomosis are directed in the normal flow direction towards the large intestine. Pancreatic juices arriving (arrows 58) from upstream in the jejunum are directed outside the tube 10 and thus away from the anastomosis 59 and also towards the large intestine, thereby reducing the possibility of reflux of these juices through the anastomosis 50.

The tube 10 may also be configured with a directional flow characteristic, to admit flow of stomach content (e.g. chyme) in a first direction through the tube 10 away from the stomach (arrow 62), and to obstruct flow in an opposite second direction through the tube 10. The one-way characteristic can also block reflux of bowel juices, for example, pancreatic juices in the jejunal loop 54 , through the anastomosis 50 towards the stomach, thereby reducing stomach discomfort for the patient.

The one-way characteristic of the tube may be implemented by at least one, optionally multiple, flaps 56 (Fig. 15) provided on the interior of the tube 10. The flaps may have a flexible and/or loose annular shape, or comprise individual arcuate segments. The flaps 56 are directed towards the outlet. Flow in the first direction (arrow 62) pushes the flaps 56 against the wall of the tube 10 to admit flow. Flow in the reverse direction tends to push the flaps 56 inwardly towards the centre of the channel, to narrow the channel in the tube 10, and hence obstruct flow. Alternatively, the tube 10 may itself be collapsible, like a sock, to perform a one-way valving function. Flow in the first direction opens and distends the tube 10. Flow in the opposite second direction collapses the tube on itself, to obstruct flow.

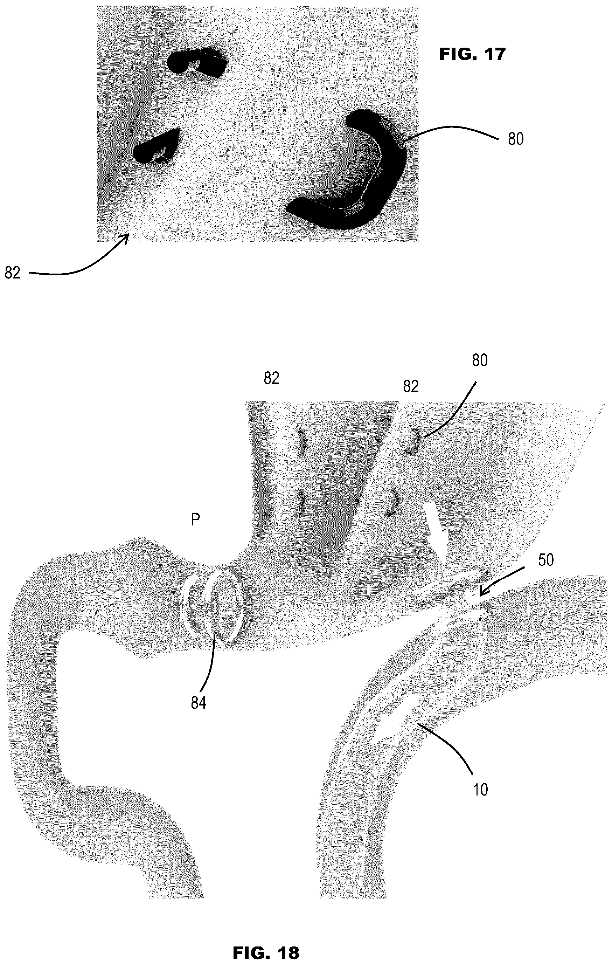

Referring to Figs. 16 and 17, a further illustrated technique for treating diabetes or obesity, involves application of at least one tissue-penetrating fixing 80 to reduce the natural volume of the stomach by creating one or more pleats 82 (or artificial folds) in the stomach wall. In the illustrated example, the fixing 80 is a staple, optionally having a U-shaped profile, with T-shaped limb ends, as described above. The fixing may, for example, be placed by an endoscopic technique or a laparoscopic technique.

The one or more pleats 82 may extend in a direction that is generally (i) from top to bottom of the stomach, and/or (ii) from the oesophagus to the pylorus. Such pleats 82 can form a generally sleeve shape cavity or passage in the stomach.

Referring to Fig. 18, the stomach reduction pleats 82 can also be used in combination with a tube 10, and optionally in combination with an anastomosis 50, for example, a gastro-jejunal anastomosis 50. The pylorus P can be closed or plugged by a closure device 84. The closure device 84 may, for example, comprise rings similar to those described for Figs. 8 to 10 above, for anchoring to the pyloric sphincter.

It will be appreciated that the above description is merely illustrative of examples of the invention, and does not limit the scope of protection. Protection is claimed for any novel feature or idea disclosed herein and/or in the drawings, whether or not emphasis has been placed thereon.

## Claims

1. An anchor (20) for anchoring a tube (10) with respect to a pylorus of a patient, the anchor comprising a gastric bulb (22) coupled or couplable to a proximal end of the tube, the gastric bulb (22) expandable from a collapsed condition for introduction into the stomach, to an operative condition within the stomach.

2. The anchor of claim 1, wherein in the expanded condition a pressure within the bulb (22) is (i) not greater than atmospheric pressure, and/or (ii) not greater than surrounding pressure within the stomach.

3. The anchor of claim 1 or 2, wherein the gastric bulb (22) is configured to partly collapse in response to stomach contractions.

4. The anchor of any preceding claim, wherein the gastric bulb (22) is self-expandable from the collapsed condition to the operative condition.

5. The anchor of claim 4, wherein the gastric bulb (22) comprises a self-expandable structure (24), the structure optionally being a frame structure and/or a mesh structure.

6. The anchor of claim 5, wherein the gastric bulb (22) has communication apertures allowing chyme to pass into the interior of the bulb.

7. The anchor of any of claims 1 to 6, wherein the gastric bulb (22) comprises a fluid-tight chamber, and an inlet port (26) for (i) allowing fluid to be drawn into the chamber as the gastric bulb expands to its operative condition, and/or (ii) admitting fluid into the chamber for inflating the gastric bulb.

8. The anchor of any preceding claim, wherein the gastric bulb (22) is (i) coupled to the tube by one or more tethers (18) and/or struts, and/or (ii) directly mounted on the tube.

9. An anchor (20) optionally according to any preceding claim, the anchor for anchoring a tube with respect to a pylorus of a patient, the anchor comprising a first ring (30) for fitting against a stomach-side of a pyloric sphincter, and a second ring (32) for fitting against a duodenal-side of the pyloric sphincter, the first and second rings (30, 32) coupled to one another for clamping the pyloric sphincter between the rings, the first and second rings (30, 32) having different inner and/or outer diameters from each other, optionally such that one ring has an inner diameter greater than the outer diameter of the other.

10. The anchor of claim 9, wherein the first and/or second rings (30, 32) are collapsible to a collapsed condition for introduction to the pylorus.

11. The anchor of claim 9 or 10, further comprising connecting elements (34) between the rings, and coupled to the rings for selectively drawing the rings closer to one another for clamping the pyloric sphincter, the connecting elements (34) optionally being filaments or sutures.

12. The anchor of claim 9, 10 or 11, further comprising at least one inner ring (38) for corralling an inner peripheral edge of the pyloric sphincter, optionally wherein the at least one inner ring comprises first and second inner rings (38a, 38b).

13. The anchor of claim 9, 10, 11 or 12, wherein the first and/or second ring comprises one or more of: metal; and/or a shape memory alloy optionally, nitinol; and/or plastics.

14. An anchor (20) optionally according to any preceding claim, the anchor for anchoring a tube with respect to a pylorus of a patient, the anchor comprising at least one resilient element (29), expandable from a collapsed condition for introduction into the stomach, and an expanded operative condition in which the resilient element has an at least partly helix shape.

15. The anchor of claim 14, wherein (i) the resilient member (29) defines a helix shape that expands in a radial direction progressively away from the proximal end of the tube; and/or (ii) the helix shape includes open space between adjacent turns or windings of the helix.

16. A tube (10) optionally comprising an anchor as defined in any preceding claim, the tube configured for insertion into the gastro-intestinal tract, the tube configured for admitting passage of stomach and/or bowel content in a first direction therethrough, and for obstructing passage of stomach and/or bowel content in an opposite second direction.

17. The tube according to claim 16, comprising at least one, optionally a plurality, of valving elements (56) configured for admitting passage of stomach and/or bowel content in the first direction therethrough, and for obstructing passage of stomach and/or bowel content in the second direction.

18. The tube according to claim 17, wherein the valving element (56) comprises a flap that opens to admit passage stomach and/or bowel content in the first direction through the tube, and closes to obstruct passage of stomach and/or bowel content in the second direction.

19. A tube (10) optionally according to any of claims 16 to 18 and/or optionally comprising an anchor as defined in any of claims 1 to 15; the tube for introduction at the duodenum of a patient, the tube made of flexible plastics and comprising a suture-permeable and/or staple-permeable layer of woven or non-woven fabric (46) near or at the proximal end, the fabric (46) permitting anchoring of the tube by suturing or stapling through the fabric into body tissue near or at the pylorus, the fabric resisting tear propagation within the plastics.

20. A tube (10) optionally according to any of claims 16 to 19 and/or optionally comprising an anchor as defined in any of claims 1 to 15; the tube for introduction into a gastro-intestinal tract of a patient, the outer surface of the tube being provided with at least one open channel (46) extending at least partly axially and/or longitudinally with respect to the tube for permitting gastric secretions to travel outside the tube, optionally wherein the channel has a shape selected from:
a helical shape around the exterior of the tube; and/or a longitudinal flute.

21. Apparatus comprising a tube (10) optionally according to any of claims 16 to 20 and/or optionally comprising an anchor as defined in any of claims 1 to 15; the tube (10) for introduction into a gastro-intestinal tract of a patient, and the apparatus comprising or further comprising at least one gastric balloon (22) configured to bear against the stomach wall away from the pylorus, optionally for inducing a feeling of satiety in a patient.

22. Apparatus according to claim 21, wherein the balloon is filled with gas to float in the stomach and/or wherein the balloon is magnetically repulsed with respect to the tube; and/or wherein the balloon is tethered to the tube and/or wherein multiple space-occupying balloons are provided for nestling together.
